# EUROPÄISCHE PATENTSCHRIFT

(11) **EP 2 313 245 B1**
(45) Veröffentlichungstag und Bekanntmachung des Hinweises auf die Patenterteilung: **25.05.2016**
(21) Anmeldenummer: 09781330.7
(22) Anmeldetag: 31.07.2009
(51) Int. Cl.: B29C 49/36, B65B 55/08, A61L 2/20, B65G 29/00, B29C 49/12, B65B 55/10, A61L 2/08, B65G 47/84

(54) **VORRICHTUNG UND VERFAHREN ZUM UMFORMEN VON KUNSTSTOFFVORFORMLINGEN MIT STERILRAUM**
APPARATUS AND PROCESS FOR SHAPING PLASTIC PREFORMS, COMPRISING A STERILE CHAMBER
DISPOSITIF ET PROCÉDÉ POUR FAÇONNER DES PRÉFORMES EN PLASTIQUE POURVU D'UNE SALLE STÉRILE

(30) Priorität: 18.08.2008 DE 102008038141
(43) Veröffentlichungstag der Anmeldung: 27.04.2011
(60) Teilanmeldung: 10194081.5
(73) Patentinhaber: Krones AG, 93073 Neutraubling (DE)
(72) Erfinder: MARTINI, Oliver, 93073 Neutraubling (DE); DAHMEN, Michael, 93073 Neutraubling (DE); ENGELHARD, Patrick, 93073 Neutraubling (DE)
(74) Vertreter: Bittner, Bernhard
(86) Internationale Anmeldenummer: PCT/EP2009/059922
(87) Internationale Veröffentlichungsnummer: WO 2010/020529

(56) Entgegenhaltungen:
- DE-A1- 1 561 985
- US-A- 4 063 867
- US-A- 4 880 581
- DATABASE WPI Week 199227 Thomson Scientific, London, GB; AN 1992-223069 XP002598598 -& JP 4 147824 A (DAINIPPON PRINTING CO LTD) 21. Mai 1992 (1992-05-21)

## Beschreibung

Die vorliegende Erfindung bezieht sich auf eine Vorrichtung und ein Verfahren zum Umformen von Kunststoffbehältnissen gemäß der Ansprüche 1 und 15. Im Bereich der getränkeherstellenden Industrie ist es bekannt, Kunststoffbehältnisse zu verwenden, wobei diese Künststoffbehältnisse durch einen Umformungsvorgang, und insbesondere einem Blasvorgang, aus Kunststoffvorformlingen erzeugt werden. Dabei sind üblicherweise Blasräder vorgesehen, an denen mehrere Blasstationen angeordnet sind, wobei innerhalb dieser Blasstationen die Kunststoffvorformlinge durch Beaufschlagung mit Druckluft gegen eine Innenwandung der entsprechenden Blasform expandiert werden.

Für viele Getränke ist es dabei erforderlich, diese unter aseptischen Bedingungen abzufüllen. Dabei ist es bekannt, dass ein Sterilisationsprozess für das aseptische Abfüllen mit der Sterilisation der bereits fertig gestellten Flaschen in einem dafür vorgesehen Reinraum beginnt. Alle im Stand der Technik davor stattfindenden Prozesse wie die Preform-Herstellung, deren Transport, deren Erwärmung und deren Blasen zur Flasche finden in einer unsterilen Umgebung statt. In diesem Falle ist es erforderlich, eine relativ große Fläche, nämlich diejenige der fertig erzeugten Kunststoffflasche, zu sterilisieren.

Daher ist es meist wünschenswert, nicht die Kunststoffflasche selbst zu sterilisieren, sondern den Kunststoffvorformling, da dieser eine erheblich geringere Oberfläche aufweist. Allerdings ist es erforderlich, die Behältnisse nach deren Sterilisation, insbesondere durchgängig, unter sterilen Bedingungen zu transportieren, zumindest bis diese verschlossen sind, um auf diese Weise eine weitere Verunreinigung der Behältnisse zu verhindern.

Die EP 0 794 903 B1 beschreibt ein System und ein Verfahren zum sterilen Verpacken von Getränken. Dabei wird ein Getränkebehälter aus einem geformten Vorformling durch Blasformgebung gebildet, anschließend wird der Behälter mit einem sterilen Getränk befüllt und schließlich der gefüllte Behälter mit einer sterilisierten Verschlusskappe gefüllt. Dabei werden unterschiedliche Sterilisationsgrade in verschiedenen Teilen der Kammer gesteuert, wobei die Sterilisationsgrade mit dem Grad korreliert sind, der für den Verfahrensschritt erforderlich ist, der in den betreffenden Kammerteil durchgeführt wird.

In diesem Herstellungsverfahren wird der gesamte Herstellungsprozess der Flasche und auch der Füll- und Verschließprozess durchgängig unter sterilen Bedingungen durchgeführt. Dabei ist auch eine vollständige Umformungseinheit der Behältnisse in einem Reinraum angeordnet. Dieses Verfahren gewährleistet einen hohen Sterilisierungs- und Reinheitsgrad der auf diese Weise befüllten Behältnissen. Andererseits ist jedoch der Aufwand für die Sterilisierung relativ hoch, da einerseits sehr große Räume steril gehalten werden müssen und andererseits auch eine Vielzahl von Maschinenteilen, namentlich im Bereich der Blasvorrichtung vorhanden sind, die steril gehalten werden müssen.

US-A-4 880 581 offenbart eine Vorrichtung und ein Verfahren zum umformen von Kunststoffbehältnissen gemäß dem Stand der Technik.

Der vorliegenden Erfindung liegt daher die Aufgabe zugrunde, den Aufwand zum Sterilisieren bzw. Sterilhalten einer Umformungseinheit, bzw. Blaseinrichtung für Behältnisse zu reduzieren. Andererseits sollten jedoch für die Behältnisse möglichst sterile Herstellungsbedingungen geschaffen werden. Dies wird erfindungsgemäß durch eine Vorrichtung nach Anspruch 1 und ein Verfahren nach Anspruch 15 erreicht. Vorteilhafte Ausführungsformen und Weiterbildungen sind Gegenstand der Unteransprüche.

Eine Vorrichtung zum Umformen von Kunststoffvorformlingen zu Kunststoffbehältnissen weist eine Transporteinrichtung auf, an der eine Vielzahl von Blasstationen angeordnet ist, wobei jede dieser Blasstationen eine Blasform aufweist, innerhalb derer ein Kunststoffvorformling zu einem Kunststoffbehältnis umformbar ist und wobei die Vorrichtung einen Reinraum aufweist, innerhalb dessen die Behältnisse transportiert werden können. Derjenige Bereich der Transporteinrichtung, an dem die Blasstationen angeordnet sind, ist in dem Reinraum angeordnet und ein weiterer Bereich der Transporteinrichtung ist außerhalb des Reinraums angeordnet.

Damit ist insbesondere bei der Vorrichtung ein Reinraumkanal vorgesehen, durch welchen hindurch die Kunststoffvorformlinge bzw. Behältnisse in den Blasstationen geführt werden und ein weiterer Bereich der Transporteinrichtung wird außerhalb des Reinraums bewegt. Damit werden Teile der Umformungseinheit wie einer Blasmaschine, z. B. das gesamte Blasrad bzw. die Blaskavitäten durch einen Reinraum bzw. abgeschlossenen Isolator von dem Rest der Blasmaschine getrennt.

Vorzugsweise handelt es sich bei der Transporteinrichtung um ein Transportrad, welches sich um eine vorgegeben Achse dreht, wobei wenigstens die Achse bzw. eine Welle der Transporteinrichtung außerhalb des Reinraums angeordnet ist. Damit wird erreicht, dass der Reinraum möglichst klein gehalten wird und damit auch das Innenvolumen des Reinraums gering gehalten werden kann. Weiterhin wird auf diese Weise auch erreicht, dass eine möglichst große Anzahl an Maschinenteilen, welche nicht unmittelbar mit den Blasstationen in Kontakt sind, außerhalb des Reinraums geführt werden können und auch auf diese Weise werden Kontaminationen gering gehalten.

Vorzugsweise weist der Reinraum wenigstens abschnittsweise ein ringförmiges Profil auf bzw. ein torusartiges Profil, wobei hier jedoch der Querschnitt dieses torusartigen Profils bevorzugt von einer Kreisform abweicht. Dies bedeutet, dass die Blasstationen durch die Transporteinrichtung auf einer im Wesentlichen kreisförmigen Bahn geführt werden.

Bei einer weiteren vorteilhaften Ausführungsform ist an jeder Blasstation eine Reckstange zum Recken der Kunststoffvorformlinge angeordnet und diese Reckstange ragt wenigstens zeitweise und abschnittsweise aus dem Reinraum heraus. Die Reckstange wird bekanntlich verwendet, um die Kunststoffvorformlinge im Rahmen des Herstellungsprozesses zu dehnen. Prinzipiell wäre es auch möglich, die gesamte Reckstange stets im Inneren des Reinraums zu führen. Zu diesem Zwecke müsste jedoch der Reinraum erheblich in seinem Volumen vergrößert werden. In der bevorzugten Ausführungsform wird daher vorgeschlagen, dass die Reckstange durch eine Öffnung in dem Reinraum nach außen ragt. Um gleichzeitig Kontaminationen dieser Reckstange zu verhindern, ist besonders bevorzugt ein Faltenbalg vorgesehen, in dessen Inneren die Reckstange verläuft, so dass die Reckstange selbst nicht mit der Außenwelt in Kontakt kommt. Innerhalb des Faltenbalgs herrschen damit auch sterile Bedingungen.

Der Reinraum ist von mehreren Wandungen begrenzt und wenigstens eine dieser Wandungen ist gegenüber einer weiteren Wandung bewegbar und insbesondere drehbar angeordnet.

Vorzugsweise ist eine radial außen angeordnete Wandung des Reinraum stationär angeordnet. Damit kann eine Wandung mit einem insbesondere zylinderförmigen Außenprofil vorgesehen sein, welche den Reinraum begrenzt. Vom Inneren dieser Wandung ist eine weitere Wandung vorgesehen, die den Reinraum zu der anderen Seite hin begrenzt und die drehbar angeordnet ist. Vorzugsweise wird diese innen angeordnete Wandung den einzelnen Blasstationen mitgedreht. Vorzugsweise liegen sich die erwähnte drehbare Wand und die stationär angeordnete Wand gegenüber. Weiterhin wird der Reinraum von einer Wandung in Form eines Deckels begrenzt, wobei dieser Deckel vorzugsweise einteilig mit der drehbaren Wand ausgebildet ist.

Vorzugsweise ist zwischen wenigstens zwei Wandung bzw. einer Wandung und einem Deckel eine Dichtungseinrichtung angeordnet. Diese Dichtungseinrichtung dichtet bevorzugt gegenüber einander bewegliche Teile gegeneinander ab. So wäre es beispielsweise möglich, zwischen einer Wandung und einem Deckel ein so genanntes Wasserschloss vorzusehen, bei dem eine hier bevorzugt ringförmiger Wasserkanal vorgesehen ist, in dem ein Abschnitt des gegenüber diesem Wasserkanal beweglichen Teils geführt wird.

Bei einer weiteren vorteilhaften Ausführungsform weist die Vorrichtung eine Zuführeinrichtung auf, um der Transporteinrichtung die Kunststoffvorformlinge zu übergeben und diese Zuführeinrichtung ist innerhalb des Reinraums angeordnet. Damit weist hier bevorzugt der Reinraum eine Ausbuchtung bzw. eine Abweichung von dem ansonsten kreisförmigen Querschnitt auf, und in dieser Ausbuchtung ist entsprechend die Zuführeinrichtung wie beispielsweise ein Transportstern für die Vorformlinge angeordnet. Auf diese Weise kann eine lückenlose Übergabe der Behältnisse von der Zuführeinrichtung an die Transporteinrichtung innerhalb eines Sterilraums erfolgen.

Bei einer weiteren vorteilhaften Ausführungsform weist die Vorrichtung auch eine Abführeinrichtung auf, um von der Transporteinrichtung die ausgebildeten Kunststoffbehältnisse zu übernehmen und diese Abführeinrichtung ist ebenfalls innerhalb des Reinraums angeordnet. Auf diese Weise kann auch beim Abtransport der Behältnisse ein Reinraum gewahrt werden. Damit ist es möglich, dass ein Einbringen von Vorformlingen aus vorgelagerten Aggregaten in den Isolator bzw. Reinraum sowie ein Ausbringen der Flaschen zu einem nächsten Aggregat unter Reinraumbedingungen möglich ist. Der Isolator bzw. Reinraum kann mit Reinigungs- und Sterilisationsmedien beaufschlagt werden.

Bei einer weiteren vorteilhaften Ausführungsform ist innerhalb des Reinraums ein Sterilgas vorgesehen und dieses Sterilgas steht unter einem Druck, der höher ist als ein Druck außerhalb des Reinraums. Damit kann der Reinraum optional durch das Einbringen von sterilisierter Luft auf einem höheren Druckniveau als die Umgebung gehalten werden, wodurch das Eindringen von Mikroorganismen verhindert werden kann. Weiterhin besteht die Möglichkeit, dem Reinraum kontinuierlich einen antimikrobiellen Wirkstoff zuzuführen und auf diese Weise ein hygienisches Umfeld aufrecht zu erhalten.

Durch einen räumlich begrenzten Reinraum in der Blasvorrichtung ist es möglich, vorher entkeimte Vorformlinge ohne Rekontamination sowohl außen als auch innen während des Streckblasvorgangs zum Füller zu transportieren. Des Weiteren ist der Reinraum im Vergleich zur gesamten Umformungsvorrichtung einfacher auf einem keimarmen Level zu halten.

Die vorliegende Erfindung ist weiterhin auf eine Anlage zum Herstellen von Kunststoffbehältnissen gerichtet, welche eine Vorrichtung zum Umformen von Kunststoffbehältnissen gemäß Anspruch 1 aufweist, sowie eine Heizeinrichtung, wobei diese Heizeinrichtung in einer Transportrichtung der Kunststoffvorformlinge stromaufwärts bezüglich der oben erwähnten Vorrichtung angeordnet ist. Diese Heizeinrichtung dient dazu, um die Vorformlinge zu erwärmen, damit diese anschließend in einem Blasvorgang zu Behältnissen expandiert werden können. Stromabwärts bzw. nach der Umformungsvorrichtung ist eine Fülleinrichtung vorgesehen, welche die Behältnisse mit einem Getränk, insbesondere mit einem aseptischen Produkt, befüllt. Auch diese Fülleinrichtung ist dabei in einem Reinraum angeordnet. Weiterhin erstreckt sich bevorzugt der Reinraum bis in den Bereich einer Verschließeinrichtung, welche die Behältnisse mit einem Verschluss verschließt.

Weiterhin weist die Anlage bevorzugt eine Sterilisationseinrichtung auf, welche wenigstens einen Bereich der Kunststoffvorformlinge vor Erreichen der Vorrichtung sterilisiert. Dabei kann diese Sterilisation mit einem gasförmigen Medium, wie insbesondere Wasserstoffperoxid, durchgeführt werden. Es wäre jedoch auch möglich, dass die Sterilisation unter Verwendung von Strahlung, wie beispielsweise Elektronenstrahlen und/oder UV-Licht bewirkt wird. Dabei ist bevorzugt eine Sterilisationseinrichtung vorgesehen, welche insbesondere auch die Innenoberfläche der Kunststoffvorformlinge sterilisiert. Daneben kann jedoch auch die Außenoberfläche der Kunststoffvorformlinge sterilisiert werden.

Bei einer weiteren vorteilhaften Ausführungsform weist die Anlage einen weiteren Reinraum auf, der in der Transporteinrichtung der Kunststoffvorformlinge vor der oben erwähnten Vorrichtung angeordnet ist. Vorzugsweise geht dieser weitere Reinraum in den Reinraum der Umformungseinrichtung über, Auf diese Weise ist es möglich, die Kunststoffbehältnisse ausgehend von deren Sterilisation kontinuierlich bis zum Verschließen zu fördern und gleichwohl die hierzu nötigen Reinräume relativ gering zu halten. Damit ist bevorzugt der Reinraum als sich von der Sterilisationseinrichtung bis zu der Verschließeinrichtung erstreckender Kanal vorgesehen, der besonders bevorzugt jeweils an die entsprechenden Blasstationen oder Halteeinrichtungen wie Greifelemente für die Vorformlinge oder Kunststoffbehältnisse angepasst ist.

Die vorliegende Erfindung ist weiterhin auf ein Verfahren zum Umformen von Kunststoffvorformlingen gemäß Anspruch 15 gerichtet, wobei die Kunststoffvorformlinge mittels einer Transporteinrichtung, an der eine Vielzahl von Blasstationen angeordnet ist, transportiert werden und während dieses Transports zu Kunststoffbehältnissen umgeformt werden. Dabei werden die Blasstationen wenigstens abschnittsweise und bevorzugt vollständig durch einen Reinraum transportiert. Erfindungsgemäß ist wenigstens ein Bereich der Transporteinrichtung außerhalb des Reinraums angeordnet. Genauer gesagt wird bevorzugt auch wenigstens ein Bereich der Transporteinrichtung außerhalb des Reinraums bewegt. Bei einem weiteren bevorzugten Verfahren werden die Blasstationen auf einer kreisförmigen Bahn bewegt und besonders bevorzugt ständig innerhalb des Reinraums bewegt.

Weitere Vorteile und Ausführungsformen ergeben sich aus den beigefügten Zeichnungen:

Darin zeigen:
- Fig. 1: eine schematische Darstellung einer Anlage zum Herstellen von Kunststoffbehältnissen; und
- Fig. 2: eine Ansicht eines Reinraums im Bereich einer Blasstation.

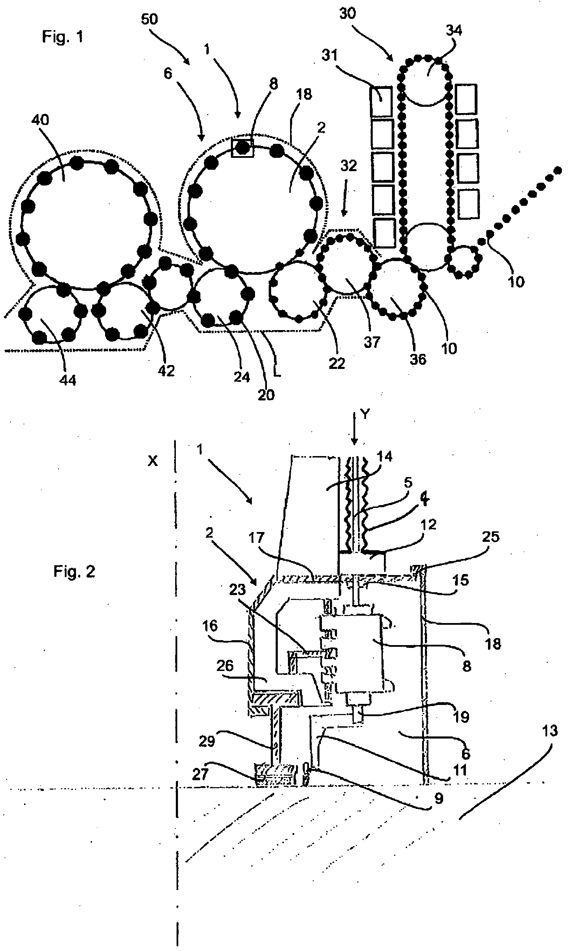

Figur 1 zeigt eine schematische Darstellung einer Anlage zum Herstellen von Kunststoffbehältnissen. Diese Anlage 50 weist eine Heizeinrichtung 30 auf, in der Kunststoffvorformlinge 10 erwärmt werden. Dabei werden diese Kunststoffvorformlinge 10 mittels einer Transporteinrichtung 34, wie hier einer umlaufenden Kette, durch diese Heizeinrichtung 30 geführt und dabei mit einer Vielzahl von Heizelementen 31 erwärmt. An diese Heizeinrichtung 30 schließt sich eine Übergabeeinheit 36 an, welche die Vorformlinge 10 an eine Sterilisationseinrichtung 32 übergibt. Diese Sterilisationseinrichtung 32 weist dabei hier ebenfalls ein Transportrad 37 auf und an diesem Transportrad 37 oder auch stationär können Sterilisationselemente angeordnet sein. In diesem Bereich ist beispielsweise eine Sterilisation durch Wasserstoffperoxidgas oder auch, wie oben erwähnt, durch elektromagnetische Strahlung möglich. Insbesondere wird in diesem Bereich eine Innensterilisation der Vorformlinge durchgeführt.

Das Bezugszeichen 6 bezeichnet in seiner Gesamtheit einen Reinraum, dessen Außenbegrenzungen hier durch die punktierte Linie L angedeutet ist. Man erkennt, dass dieser Reinraum 6 in dem Bereich der Sterilisationseinheit 32 beginnt. In diesem Bereich können Schleuseneinrichtungen vorgesehen sein, um die Kunststoffvorformlinge in den Reinraum 6 einzuführen, ohne dass dabei zu viel Gas innerhalb des Reinraums verloren geht.

Der Reinraum ist, wie durch die gestrichelte Linie L angedeutet, an die Außengestalt der einzelnen Anlagenkomponenten angepasst. Auf diese Weise kann das Volumen des Reinraums reduziert werden.

Das Bezugszeichen 1 bezeichnet in ihrer Gesamtheit eine Umformungsvorrichtung, bei der an einem Transportrad 2 eine Vielzahl von Blasstationen 8 angeordnet ist, wobei hier lediglich einer dieser Blasstationen 8 dargestellt ist. Mit diesen Blasstationen 8 werden die Kunststoffvorformlinge 10 zu Behältnissen 20 expandiert. Obwohl hier nicht detailliert gezeigt, befindet sich nicht der gesamte Bereich des Transporteinrichtung 2 innerhalb des Reinraums 6, sondern der Reinraum 6 bzw. Isolator ist gewissermaßen als Mini-Isolator innerhalb der gesamten Vorrichtung realisiert. So wäre es möglich, dass der Reinraum zumindest im Bereich der Umformungsvorrichtung 1 kanalartig ausgeführt ist.

Das Bezugszeichen 22 bezieht sich auf eine Zuführeinrichtung, welche die Vorformlinge an die Umformungseinrichtung 1 übergibt und das Bezugszeichen 24 auf eine Abführeinrichtung, welche die hergestellten Kunststoffbehältnisse 20 von der Umformungsvorrichtung 1 abführt. Man erkennt, dass der Reinraum 6 in dem Bereich der Zuführeinrichtung 22 und der Abführeinrichtung 24 an jeweils Ausnehmungen aufweist, welche diese Einrichtungen 22, 24 aufnehmen. Auf diese Weise kann in besonders vorteilhafter Weise eine Übergabe der Kunststoffvorformlinge 10 an die Umformungsvorrichtung 1 bzw. eine Übernahme der Kunststoffbehältnisse 20 von der Umformungsvorrichtung 1 erreicht werden.

Mit einer Übergabeeinheit 42 werden die expandierten Kunststoffbehältnisse an eine Befüllungseinrichtung 40 übergeben und von dieser Befüllungseinrichtung 40 anschließend über eine weitere Transporteinheit 44 abgeführt. Dabei befindet sich auch die Befüllungseinrichtung 40 innerhalb des besagten Reinraums 6. Auch im Falle der Befüllungseinrichtung wäre es möglich, dass nicht die gesamte Befüllungseinrichtung 40 mit beispielsweise einem Reservoir für ein Getränk vollständig innerhalb des Reinraums 6 angeordnet sind, sondern auch hier lediglich diejenigen Bereiche, in denen tatsächlich die Behältnisse geführt werden. Insoweit könnte auch die Befüllungseinrichtung in ähnlicher Weise aufgebaut sein wie die Vorrichtung 1 zum Umformen von Kunststoffvorformlingen 10.

Wie erwähnt, ist der Reinraum 6 im Bereich der Vorrichtung 1 auf einen geringst möglichen Bereich reduziert, nämlich im Wesentlichen auf die Blasstationen 8 selbst. Durch diese kleinbauende Gestaltung des Reinraums 6 ist es leichter und schneller möglich, einen Reinraum überhaupt herzustellen und auch die Sterilhaltung in der Betriebsphase ist weniger aufwändig. Auch wird weniger Sterilluft benötigt, was zu kleineren Filteranlagen führt und auch die Gefahr von unkontrollierter Wirbelbildung wird reduziert.

Figur 2 zeigt eine Detaildarstellung der Vorrichtung 1 im Bereich einer Blasstation 8. Eine Vielzahl derartiger Blasstationen 8 wird mit einer Transporteinrichtung 2 drehend um eine Achse X bewegt. Die Blasstation 8 ist, wie in Figur 2 ersichtlich, innerhalb des Reinraums 6, der hier kanalartig ausgebildet ist, geführt. Dieser Reinraum 6 wird abgeschlossen durch eine bewegliche Seitenwand 16 und einen einteilig mit dieser Seitenwand 16 ausgebildeten Deckel 17. Diese Seitenwand 16 und der Deckel 17 drehen dabei mit der Blasstation 8 mit.

Das Bezugszeichen 18 bezieht sich auf eine weitere Wandung, welche den Reinraum 16 begrenzt. Diese Wandung 18 ist hier eine außen liegende Wandung, welche stationär angeordnet ist. Zwischen dem Deckel 17 und der Wandung 18 ist eine Dichtungseinrichtung 25 vorgesehen, welche die gegeneinander beweglichen Elemente 17 und 18 gegeneinander abdichtet, beispielsweise, wie oben erwähnt, unter Verwendung eines Wasserschlosses. Der untere Bereich der Wandung 18 ist fest und abdichtend an einem Boden 13 angeordnet. Innerhalb des Reinraums 6 und hier unmittelbar an der Wandung 16 anliegend ist ein Träger 26 vorgesehen, der sich ebenfalls drehend bewegt und an dem wiederum eine Halteeinrichtung 23 vorgesehen ist, welche die Blasstation 8 hält.

Das Bezugszeichen 11 bezieht sich auf eine Folgeeinrichtung, welche von einer Führungskurve 9 betätigt werden kann, um die Blasstation auf ihrem Weg durch den Reinraum 6 zu öffnen und zu schließen, um insbesondere den Kunststoffvorformling in die Blasstation einzulegen und um ihn auch wieder zu entnehmen. Dabei ist eine Führungskurve 9 auch innerhalb des Reinraums 6 angeordnet. Es wäre jedoch beispielsweise auch möglich, etwa bereits einen Abschnitt 19 unterhalb der einzelnen Blasstationen 8 aus dem Reinraum 6 herauszuführen.

Die Transporteinrichtung 2 kann noch weitere Elemente aufweisen, welche oberhalb des Reinraums 6 angeordnet sind.

Der Träger 26 ist dabei fest auf einem Haltekörper 29 angeordnet und dieser Haltekörper ist wiederum beweglich gegenüber dem Boden 13. Dabei bezieht sich das Bezugszeichen 27 auf eine weitere Dichtungseinrichtung, welche auch in diesem Bereich eine Abdichtung der gegeneinander beweglichen Bereiche 13 und 29 bewirkt.

Das Bezugszeichen 5 bezieht sich auf eine Reckstange, welche gegenüber der Blasstation beweglich ist, um die Kunststoffvorformlinge 10 in ihrer Längsrichtung zu recken. Dabei ist auf dem Deckel 17 hier ein Schlitten 12 angeordnet, demgegenüber die Reckstange in der Richtung Y beweglich ist. Das Bezugszeichen 14 bezieht sich auf eine weitere Halterung für diesen Schlitten 12 der Reckstange 5.

Man erkennt, dass bestimmte Bereiche der Reckstange während des Blasvorgangs sowohl außerhalb des Reinraums 6 als auch innerhalb des Reinraums sind. Zu diesem Zweck ist es möglich, außerhalb des Reinraums 6 bzw. oberhalb des Schlittens 12 eine Schutzeinrichtung 4 wie einen Faltenbalg vorzusehen, der die Reckstange 5 umgibt, so dass kein Bereich der Reckstange 5 unmittelbar mir der Außenumgebung in Berührung kommt.

## Patentansprüche

1. Vorrichtung (1) zum Umformen von Kunststoffvorformlingen (10) zu Kunststoffbehältnissen (20) mit einer Transporteinrichtung (2), an der eine Vielzahl von Blasstationen (8) angeordnet ist, wobei jede dieser Blasstationen (8) eine Blasform aufweist, innerhalb derer ein Kunststoffvorformling (10) zu einem Kunststoffbehältnis (20) umformbar ist, wobei die Vorrichtung (1) einen Reinraum (6) aufweist, innerhalb dessen die Kunststoffvorformlinge (10) transportiert werden können, wobei derjenige Bereich der Transporteinrichtung (2), an dem die Blasstationen (8) angeordnet sind, in dem Reinraum (6) angeordnet ist und wenigstens ein weiterer Bereich der Transporteinrichtung (2) außerhalb des Reinraums (6) angeordnet ist, wobei der Reinraum (6) von mehreren Wandungen (16, 17, 18) begrenzt ist und wenigstens eine dieser Wandungen (16, 17, 18) gegenüber einer weiteren Wandung (16, 17, 18) bewegbar ist.

2. Vorrichtung (1) nach Anspruch 1, **dadurch gekennzeichnet, dass** die Transporteinrichtung (2) ein Transportrad aufweist, welches sich um eine vorgegebene Achse (X) dreht, wobei wenigstens die Achse (X) außerhalb des Reinraums (6) angeordnet ist.

3. Vorrichtung (1) nach wenigstens einem der vorangegangenen Ansprüche, **dadurch gekennzeichnet, dass** der Reinraum (6) wenigstens abschnittsweise ein ringförmiges Profil aufweist.

4. Vorrichtung (1) nach wenigstens einem der vorangegangenen Ansprüche, **dadurch gekennzeichnet, dass** an jeder Blasstation (8) eine Reckstange (5) zum Recken der Kunststoffvorformlinge (10) angeordnet ist und diese Reckstange (10) wenigstens zeitweise und abschnittsweise aus dem Reinraum (6) herausragt, wobei bevorzugt dieser herausragende Abschnitt der Reckstange von einer Schutzeinrichtung (4) umschlossen ist.

5. Vorrichtung (1) nach wenigstens einem der vorangegangenen Ansprüche, **dadurch gekennzeichnet, dass** der Reinraum (6) von mehreren Wandungen (16, 17, 18) begrenzt ist und wenigstens eine dieser Wandungen (16) gegenüber einer weiteren Wandung (18) drehbar angeordnet ist.

6. Vorrichtung (1) nach Anspruch 5, **dadurch gekennzeichnet, dass** eine radial außen angeordnete Wandung (18) des Reinraums (6) stationär angeordnet ist.

7. Vorrichtung (1) nach Anspruch 5, **dadurch gekennzeichnet, dass** eine radial innen angeordnete Wandung (16) des Reinraums beweglich ist.

8. Vorrichtung (1) nach wenigstens einem der vorangegangenen Ansprüche 5 - 7, **dadurch gekennzeichnet, dass** zwischen wenigstens zwei Wandungen (17, 18) eine Dichtungseinrichtung (25) angeordnet ist.

9. Vorrichtung (1) nach wenigstens einem der vorangegangenen Ansprüche, **dadurch gekennzeichnet, dass** die Vorrichtung (1) eine Zuführeinrichtung (22) aufweist, um der Transporteinrichtung (2) die Kunststoffvorformlinge (10) zu übergeben und diese Zuführeinrichtung (22) innerhalb des Reinraums (6) angeordnet ist.

10. Vorrichtung (1) nach wenigstens einem der vorangegangenen Ansprüche, **dadurch gekennzeichnet, dass** die Vorrichtung (1) eine Abführeinrichtung (24) aufweist, um von der Transporteinrichtung (2) die Kunststoffbehältnisse (20) zu übernehmen und diese Abführeinrichtung (24) innerhalb des Reinraums (6) angeordnet ist.

11. Vorrichtung (1) nach wenigstens einem der vorangegangenen Ansprüche, **dadurch gekennzeichnet, dass** innerhalb des Reinraums (6) ein Sterilgas vorgesehen ist und dieses Sterilgas unter einem Druck steht, der höher ist als ein Druck außerhalb des Reinraums (6).

12. Anlage (50) zum Herstellen von Kunststoffbehältnissen (20) mit einer Vorrichtung (1) nach wenigstens einem der vorangegangenen Ansprüche und mit einer Heizeinrichtung (30), wobei diese Heizeinrichtung (30) an einer Transportrichtung (34) der Kunststoffvorformlinge stromaufwärts bezüglich der Vorrichtung (1) angeordnet ist.

13. Anlage (50) nach Anspruch 11, **dadurch gekennzeichnet, dass** die Anlage eine Sterilisationseinrichtung (32) aufweist, welche wenigstens einen Bereich der Kunststoffvorformlinge (10) vor Erreichen der Vorrichtung (1) sterilisiert.

14. Anlage (50) nach wenigstens einem der vorangegangenen Ansprüche, **dadurch gekennzeichnet, dass** die Anlage (50) einen weiteren Reinraum (6) aufweist, der in der Transportrichtung der Kunststoffvorformlinge vor der Vorrichtung (1) angeordnet ist.

15. Verfahren (1) zum Umformen von Kunststoffvorformlingen zur Kunststoffbehältnissen (20), wobei die Kunststoffvorformlinge (10) mittels einer Transporteinrichtung (2), an der eine Vielzahl von Blasstationen (12) angeordnet ist, transportiert werden und während dieses Transports zu Kunststoffbehältnissen (20) umgeformt werden, wobei die Blasstationen (8) wenigstens abschnittsweise durch einen Reinraum (6) transportiert werden, wobei der Reinraum (6) von mehreren Wandungen (16, 17, 18) begrenzt ist und wenigstens eine dieser Wangungen (16, 17, 18) gegenüber einer weiteren Wandung (16, 17, 18) bewegbar ist, wobei wenigstens ein Bereich der Transporteinrichtung (2) außerhalb des Reinraums (6) angeordnet ist.

## Claims

1. An apparatus (1) for shaping plastics-material pre-forms (10) into plastics-material containers (20) with a transport device (2) on which a plurality of blow-moulding stations (8) are arranged, wherein each of these blow-moulding stations (8) has a blow mould inside which a plastics-material pre-form (10) is capable of being shaped into a plastics-material container (20), wherein the apparatus (1) comprises a clean room (6) inside which the plastics-material pre-forms (10) can be transported, wherein that region of the transport device (2) on which the blow-moulding stations (8) are arranged is arranged in the clean room (6) and at least a further region of the transport device (2) is arranged outside the clean room (6), wherein the clean room (6) is limited by means of several walls (16, 17, 18) and at least one of these walls (16, 17, 18) is capable of being moved with respect to a further wall (16, 17, 18).

2. An apparatus (1) according to claim 1, **characterized in that** the transport device (2) comprises a transport wheel which rotates about a predefined axis (X), wherein at least the axis (X) is arranged outside the clean room (6).

3. An apparatus (1) according to at least one of the preceding claims, **characterized in that** the clean room (6) has an annular profile at least in sections.

4. An apparatus (1) according to at least one of the preceding claims, **characterized in that** a stretch rod (5) for stretching the plastics-material pre-forms (10) is arranged at each blow-moulding station (8), and this stretch rod (5) projects at least temporarily and in sections out of the clean room (6), wherein in a preferred manner this projecting portion of the stretch rod is surrounded by a protective device (4).

5. An apparatus (1) according to at least one of the preceding claims, **characterized in that** the clean room (6) is limited by several walls (16, 17, 18) and at least one of these walls (16) is arranged so as to be rotatable with respect to a further wall (18).

6. An apparatus (1) according to claim 5, **characterized in that** a wall (18) of the clean room (6) arranged radially on the outside is arranged in a stationary manner.

7. An apparatus (1) according to claim 5, **characterized in that** a wall (16) of the clean room arranged radially on the inside is movable.

8. An apparatus (1) according to at least one of the preceding claims 5 to 7, **characterized in that** a sealing device (25) is arranged between at least two walls (17, 18).

9. An apparatus (1) according to at least one of the preceding claims, **characterized in that** the apparatus (1) comprises a supply device (22) in order to pass the plastics-material pre-forms (10) on to the transport device (2), and this supply device (22) is arranged inside the clean room (6).

10. An apparatus (1) according to at least one of the preceding claims, **characterized in that** the apparatus (1) has a removal device (24) in order to take up the plastics-material containers (20) from the transport device (2), and this removal device (24) is arranged inside the clean room (6).

11. An apparatus (1) according to at least one of the preceding claims, **characterized in that** a sterile gas is provided inside the clean room (6) and this sterile gas is under a pressure which is higher than a pressure outside the clean room (6).

12. A plant (50) for producing plastics-material containers (20) with an apparatus (1) according to at least one of the preceding claims and with a heating device (30), wherein this heating device (30) is arranged upstream with respect to the apparatus (1) in a transport direction (34) of the plastics-material pre-forms.

13. A plant (50) according to claim 11, **characterized in that** the plant has a sterilization device (32) which sterilizes at least one region of the plastics-material pre-forms (10) before they reach the apparatus (1).

14. A plant (50) according to at least one of the preceding claims, **characterized in that** the plant (50) has a further clean room (6) which is arranged in front of the apparatus (1) in the transport direction of the plastics-material pre-forms.

15. A method (1) of shaping plastics-material pre-forms into plastics-material containers (20), wherein the plastics-material pre-forms (10) are transported by means of a transport device (2), on which a plurality of blow-moulding stations (8) are arranged, and are shaped into plastics-material containers (20) during this transport, wherein the blow-moulding stations (8) are transported at least in sections through a clean room (6), wherein the clean room (6) is limited by several walls (16, 17, 18) and at least one of these walls (16, 17, 18) is capable of being moved with respect to a further wall (16, 17, 18), wherein at least one region of the transport device (2) is arranged outside the clean room (6).

## Revendications

1. Dispositif (1) destiné à transformer des préformes en matière plastique (10) pour former des contenants en matière plastique (20), comprenant un système de transport (2) sur lequel sont agencés une pluralité de postes de soufflage (8), chacun de ces postes de soufflage (8) comprenant un moule de soufflage à l'intérieur duquel une préforme en matière plastique (10) peut être transformée pour obtenir un contenant en matière plastique (20), le dispositif (1) comprenant une salle blanche (6) à l'intérieur de laquelle les préformes en matière plastique (10) peuvent être transportées, la zone du système de transport (2) sur laquelle les postes de soufflage (8) sont agencés étant située dans la salle blanche (6) et au moins une autre zone du système de transport (2) étant située à l'extérieur de la salle blanche (6), la salle blanche (6) étant délimitée par plusieurs parois (16, 17, 18) et au moins une de ces parois (16, 17, 18) étant mobile par rapport à une autre paroi (16, 17, 18).

2. Dispositif (1) selon la revendication 1, **caractérisé en ce que** le système de transport (2) comprend un chemin de transport, lequel tourne autour d'un axe (X) prédéfini, au moins l'axe (X) étant situé à l'extérieur de la salle blanche (6).

3. Dispositif (1) selon au moins l'une des revendications précédentes, **caractérisé en ce que** la salle blanche (6) présente au moins en sections un profil annulaire.

4. Dispositif (1) selon au moins l'une des revendications précédentes, **caractérisé en ce qu'**une barre d'étirage (5) destinée à étirer les préformes en matière plastique (10) est agencée sur chaque poste de soufflage (8) et cette barre d'étirage (10) fait saillie, au moins par intermittence et au moins en sections, de la salle blanche (6), cette section en saillie de la barre d'étirage étant de préférence entourée par un système de protection (4).

5. Dispositif (1) selon au moins l'une des revendications précédentes, **caractérisé en ce que** la salle blanche (6) est délimitée par plusieurs parois (16, 17, 18) et au moins l'une de ces parois (16) est montée en rotation par rapport à une autre paroi (18).

6. Dispositif (1) selon la revendication 5, **caractérisé en ce qu'**une paroi (18) de la salle blanche (6), laquelle paroi est agencée radialement à l'extérieur, est montée fixe.

7. Dispositif (1) selon la revendication 5, **caractérisé en ce qu'**une paroi (16) de la salle blanche, laquelle paroi est agencée radialement à l'intérieur, est mobile.

8. Dispositif (1) selon au moins l'une des revendications précédentes 5 à 7, **caractérisé en ce qu'**un système d'étanchéité (25) est agencé entre au moins deux parois (17, 18).

9. Dispositif (1) selon au moins l'une des revendications précédentes, **caractérisé en ce que** le dispositif (1) comprend un système d'amenée (22) destiné à transférer les contenants en matière plastique (10) sur le système de transport (2) et ce dispositif d'amenée (22) est agencé à l'intérieur de la salle blanche (6).

10. Dispositif (1) selon au moins l'une des revendications précédentes, **caractérisé en ce que** le dispositif (1) comprend un système d'évacuation (24) destiné à réceptionner les contenants en matière plastique (20) du système de transport (2) et ce système d'évacuation (24) est agencé à l'intérieur de la salle blanche (6).

11. Dispositif (1) selon au moins l'une des revendications précédentes, **caractérisé en ce qu'**un gaz stérile est prévu à l'intérieur de la salle blanche (6) et ce gaz stérile se trouve à une pression qui est supérieure à une pression située à l'extérieur de la salle blanche (6).

12. Installation (50) destinée à produire des contenants en matière plastique (20), comprenant un dispositif (1) selon au moins l'une des revendications précédentes, et un système de chauffage (30), ce système de chauffage (30) étant agencé sur un système de transport (34) des préformes en matière plastique en amont du dispositif (1).

13. Installation (50) selon la revendication 11, **caractérisée en ce que** l'installation comprend un système de stérilisation (32), lequel stérilise au moins une zone des préformes en matière plastique (10) avant que celles-ci n'atteignent le dispositif (1).

14. Installation (50) selon au moins l'une des revendications précédentes, **caractérisée en ce que** l'installation (50) comprend une autre salle blanche (6) qui est agencée dans la direction de transport des préformes en matière plastique devant le dispositif (1).

15. Procédé (1) destiné à transformer des préformes en matière plastique pour obtenir des contenants en matière plastique (20), les préformes en matière plastique (10) étant transportées au moyen d'un système de transport (2) sur lequel une pluralité de postes de soufflage (12) sont agencés, et étant transformées pendant ce transport pour former des contenants en matière plastique (20), les postes de soufflage (8) étant transportés au moins en sections à travers une salle blanche (6), la salle blanche (6) étant délimitée par plusieurs parois (16, 17, 18) et au moins l'une de ces parois (16, 17, 18) étant mobile par rapport à une autre paroi (16, 17, 18), au moins une zone du système de transport (2) étant agencée à l'extérieur de la salle blanche (6).
